Europäisches Patentamt

European Patent Office

Office européen des brevets

(19)

(11) Publication number: **0 231 632**
**A2**

(12) **EUROPEAN PATENT APPLICATION**

(21) Application number: **86309896.8**

(22) Date of filing: **18.12.86**

(51) Int. Cl.⁴: **A61L 2/18**

(30) Priority: **20.12.85 GB 8531384**

(43) Date of publication of application:
**12.08.87 Bulletin 87/33**

(84) Designated Contracting States:
**AT BE CH DE ES FR GB GR IT LI NL SE**

(71) Applicant: **UNILEVER PLC**
**Unilever House Blackfriars P.O. Box 68**
**London EC4P 4BQ(GB)**
**GB**
Applicant: **UNILEVER NV**
**Burgemeester s'Jacobplein 1 P.O. Box 760**
**NL-3000 DK Rotterdam(NL)**
**BE CH DE ES FR GR IT LI NL SE AT**

(72) Inventor: **Groenewegen, Dirk**
**Lichtegaarde 30**
**NL-3436 ZV Nieuwegein(NL)**
Inventor: **Hall, Philip John**
**16 Somerville Drive Pound Hill**
**Crawley Sussex RH10 3JT(GB)**
Inventor: **Jones, Martin Vincent**
**31 High Street**
**Stanwick Northampton NN9 6QA(GB)**

(74) Representative: **Gambell, Derek et al**
**UNILEVER PLC Patents Division P.O. Box 68**
**Unilever House**
**London EC4P 4BQ(GB)**

(54) **Sanitizing process.**

(57) Performic acid is used as an industrial sanitizer, especially in the food industry. Preferably, this use comprises reacting a pair of precursors to form the peracid which is then applied to the surface to be sanitized, either substantially immediately or within a period before degradation results in a peracid concentration below that which is microbiocidally effective.

EP 0 231 632 A2

## SANITIZING PROCESS

The present invention relates to industrial sanitization of surfaces particularly, but not exclusively in the food industry, as well as in industrial fabric washing.

Industrial sanitization commonly involves heat treatments, the use of disinfectant (microbiocidal) substances, or both. Amongst these disinfectants are peroxygen compounds such as hydrogen peroxide and peroxy acids (peracids) such as peracetic acid.

The bactericidal and sporicidal disinfectant properties of performic, peracetic and perproprionic acids have been described by V. Merka et al in J. Hyg. Epidem. Microbiol. Immunol. 1965 (IX) 220. There it was reported that all had very similar disinfectant properties. V. Merka and J. Dvorak in the same journal, 1968 - (12) 115 gave results of an investigation into the fungicidal (mycocidal) properties of these acids. The concluded that performic acid demonstrated somewhat superior results but that this effect was not pronounced and was probably due to presence of residual formic acid from which the peracid had been prepared. Successful pre-operative sterilization of surgeon's hands using performic acid was noted by D. Kozarov et al in the same journal, 19 (1975), No. 3, 389 and the authors proposed the use of this peracid for the disinfection of surfaces and objects.

However, since then, performic acid has received no serious attention as a commercial disinfectant, probably for a number of reasons. First, the very great instability of the substance relative to the higher alkyl peracids and second, the dangerous (explosive) nature of the substance at concentrations at which it would have to be manufactured, stored and transported before dilution prior to use. These properties, combined with the perceived lack of particular advantage in choosing performic out of the other peracids, lead to concentration on use of peracetic, and sometimes perproprionic acid as the agent of choice, usually combined with a stabilizer to inhibit degradation. For example, such use of the latter two acids is described in patent specification GB 1,561,680.

Nevertheless, in view of the often large-scale character of industrial santization, for example in food process cleaning, there is a continued aim for agents of increased sanitizing power which are suitable for application in this area of industry, are environmentally acceptable and allow greater economy on application and/or lower temperatures in the sanitizing step.

Surprisingly, we have now found that in fact, of the peracids, performic acid has an exceptionally advantageous degree and range of microbiocidal properties, so that it is an especially useful sanitizer. However, its instability is a serous drawback if one wanted to use it as a disinfectant, in for example, the domestic home environment.

Thus, the essence of the present invention resides in the industrial use of performic acid as a sanitizer. By this is meant, the use of performic acid in any industrial application, including manufacturing, processing, packaging, catering and the like and of course excludes use in the home by a private individual, not for commercial purposes, or experimental use.

We have found that performic acid at relatively low concentrations and low temperatures provides a powerful and economical sanitizing agent effective against a wide range of spores, vegetative micro-organisms as well as dairy bacteriophages, and allows easy removal without leaving harmful residues on the surfaces to be sanitized.

Especially preferred, are applications in the food industry, including sanitizing (disinfection) of surfaces on which food is handled, processed or prepared, or surfaces of implements used for food handling.

Another preferred application in the food industry is the cleaning-in-place (CIP) of the insides of equipment in dairies, such as ultra-high temperature (UHT) treatment equipment, and in breweries, such as bright beer tanks.

Yet another preferred application in the food industry or elsewhere is in the sanitization of packaging material prior to use in aseptic packaging operations.

Outside the food industry, the present invention may be employed in industrial fabric washing, for example in continuous (e.g. tunnel) washing machines. Here, as well as soil removal, one aim is disinfection of the fabrics.

This especially so in the case of hospital laundries. In the fabric washing context, a further advantage is obtained from the inherent bleaching properties of the performic acid.

In general, for most industrial applications, the surface to be sanitized will be a so-called 'hard surface'. Hard surfaces for the purpose of this invention include the usual hard surfaces such as steel, wood, plastics and brick, as well as relatively soft packaging materials and e.g fruit skin. However, the present invention does not exclude admixture of the performic acid with other substances or compositions to reduce the number of micro-organisms in them to a safe level.

In the food industry, the necessity of sanitization is well known. For example, it is well recognised that in food processing and packaging operations cleaning, disinfection and sanitization are essential if edible food materials are to be produced under safe hygienic conditions.

According to a commonly used methodology (including in CIP), cleanliness of food processing equipment can be achieved by rinsing with clean water, preferably including inorganic alkalis, surface-active agents, alkaline polyphosphates and the like. In the physical removal of soils, large numbers of contaminating micro-organisms will also be removed. Nevertheless, significant numbers of micro-organisms will remain on the equipment surfaces after cleaning and these must be destroyed if the risk of contamination is to be avoided. Accordingly, a sanitizing step is required to achieve both physical and microbiological cleanliness. Also, in the processing of packaging materials for food products it is often necessary or required to include a sanitizing or sterilizing step before filling and further handling of the products (aseptic packaging).

In view of the relative instability of performic acid solutions due to the spontaneous and/or metal-ion catalysed loss of oxygen, it is very much preferred that the performic acid solution is applied to the hard surface to be sanitized simultaneously with, or within relatively shortly after its preparation. Dependent on concentration and storage temperature, it is necessary to apply the performic acid solution within a period of up to 3 days after the mixing of reactants to initiate its preparation.

When prepared and stored at ambient temperature, the performic acid solution is preferably applied within a period of from 15 minutes up to 48 hours after mixing the reactants. At higher temperatures there is a considerable reduction in reaction time and storage time after which a significant level of activity is still present.

At reaction temperatures above 45°C, the performic acid solutions can best be applied substantially directly after equilibrium has been reached. Alternatively, the acid may be prepared and stored at around or below ambient temperature and then heated just prior to use, or mixed with a hot diluent.

The present invention therefore also provides an industrial process for sanitizing a surface by preparing performic acid and applying it at an effective concentration to the surface to be sanitized, before, during or after an industrial operation.

In this context, 'industrial operation' includes manufacturing, processing and packaging operations, although others can readily be envisaged, including as described elsewhere in this specification. In most cases, the preparation of the performic acid will take place on the same site as where the industrial operation is being performed. Preferably, such on-site preparation will be effected in a reaction vessel located adjacent to the surface to be sanitized. When application takes place substantially after mixing of reactants to initiate preparation, the performic acid is preferably conveyed to the surface via a direct link, for example tubing, piping or other ducting, although of course this may contain valves, gates and the like. In another preferred realisation of the invention, the performic acid is stored in the reaction vessel prior to application. Alternatively, it may be stored in a separate storage vessel prior to use. This separate vessel may or may not be connected to the reaction vessel and/or lead to the surface to be sanitized, via a direct link (coupling), as desired.

We have also found it preferable that the performic acid should be applied to the surface within the time periods we indicate above. Also, as will be elaborated further hereinbelow, it is preferred to apply the performic acid in the form of a solution thereof. In any event, subsequent to application, in most situations it is advantageous to remove the performic acid from the surface, for example by rinsing and/or heating and/or vibration.

The most convenient way of preparing performic acid is by the conventional method of mixing solutions of hydrogen peroxide and formic acid at suitable concentrations, optionally in the presence of small amounts of catalysts, such as strong acids, e.g. sulphuric acid.

It will be appreciated that the latter method employs solutions of reactants rather that the undiluted reagents for reasons of stability and hence commercial availability. Thus, the performic acid is prepared in the form of a solution. Preferably, this solution is prepared at a concentration in the range of up to 10% by weight. Although higher concentrations are possible, too many safety hazards arise from performic acid concentrations entering the denotable region. Preferably, the performic acid solution is prepared at a concentration range being in the range of from 2 to 8% by weight, the optimal concentration range being from 3 to 7% by weight.

As will be explained further hereinbelow, the solution so prepared may be further diluted prior to its application to the surface to be sanitized. This is for reasons of economy whilst maintaining an effective working concentration. Unless the context requires otherwise, any reference herein to dilution or to solutions refers to use or presence of one or more diluents or solvents compatible with the performic acid, the preparative apparatus, and/or the surface(s) to be sanitized, as appropriate. However, it will generally be preferred to use water for reasons of economy and simplicity.

3

In general, hydrogen peroxide solutions having concentrations in the range of from 10 to 50% by weight are used, which are mixed with formic acid solutions in a wide range of concentrations of from 5 to 100% by weight. Preferably, 20-40% hydrogen peroxide solutions are mixed with 30-90% formic acid solutions, most preferably from 50 to 85% resulting in performic acid concentrations in the hereabove described optimal ranges. We have found that best results are obtained when the solutions are mixed such that in final solution, the weight ratio of the hydrogen peroxide per se component to the formic acid per se is from 1:6 to 1:1.5, preferably around 1:4.

To accelerate the establishment of equilibrium after mixing of the hydrogen peroxide and the performic acid solutions, it is often preferred to include a catalyst. As mentioned above, a suitable catalyst is sulphuric acid, preferably in a concentration up to 5% by weight, especially of from 0.5 to 3% by weight. The catalyst is preferably added to the formic acid solution prior to mixing with the hydrogen peroxide solution, although it is also possible to prior-add the catalyst to the peroxide.

The degree of dilution of the performic acid solutions to suitable working concentrations, prior to application, is dependent on the degree of sanitizing or disinfection required and the particular field of application.

Thus for example, in the area of aseptic packaging where sterilization of the packaging material is required before filling, performic acid solution will be used in working concentrations of from 1 to 100% by weight, preferably of from 5 to 20%.

When sanitizing during CIP of food processing equipment, much higher dilutions may be used. Working performic acid concentrations of from 2 to 300 ppm have been found suitable, concentrations of from 30 to 150 ppm being preferred.

In addition to the performic acid sanitizing agent, also other accessory ingredients conventionally employed in cleaning and sanitizing operations may be included, such as detergent actives (surfactants), descalers, stabilizers, corrosion inhibitors and the like. These accessory ingredients will be of any kind known to those skilled in the art. However, they should be compatible with the performic acid and be suitable for use at acid pH range of from about 1 to 4. Any accessory ingredients independently may be present in one or both of the reactants, or be added during reaction, at any subsequent stage of dilution or by mixing with the peracid solution just prior to application. Alternatively, as is common in CIP practice, such other ingredients may be applied to the surface to be sanitized, optionally followed by rinsing, before a santization step.

Suitable detergent active (surfactant) agents for this purpose include those of the anionic, cationic, non-ionic, zwitterionic and amphoteric kinds. However, the non-ionics are most preferred, especially those which are generally termed 'low-foaming'. Suitable descaling agents comprise organic and/or inorganic acids.

Additionally, the process of the present invention may comprise a step of "passivation" of any metal surface into which the peracid will come into contact, such as in the reaction vessel, auxiliary piping or (in the case of CIP), the surface to be sanitized. This is to inhibit degradation of the peracid by surface effects and/or metal-catalysed decomposition. Thus for example, stainless steel surfaces may be pre-treated by rinsing with dilute nitric acid.

In general, the sanitizing effectiveness of performic acid is sufficient to render elevated temperatures at application unnecessary. Accordingly, the performic acid solutions are applied to the hard surface at ambient temperature or, if convenient, at the operational temperature of the equipment to be cleaned. The performic acid suitably can be applied in the temperature range of from close to freezing point, corresponding to the equipment operation temperature in e.g. breweries, up to 80°C which corresponds to the operational temperature in e.g. dairy processing.

The period of time the hard surface is contacted with the performic acid solution to obtain sufficient sanitizing (contact time) is dependent on performic acid concentration, temperature and type of operation.

In the aseptic packaging field, where the packaging materials are contacted with performic acid solution, working concentrations in the range of 5-20% (generally in the form of a spray or bath), contacting may for example be only for a period of 1 to 15 seconds. After this period the packaging material is dried, optionally by way of hot air.

In equipment cleaning (including CIP), contact times are generally much longer and lie in the range of from 1 to 30 minutes, but may even be so long as overnight. For optimal results, contact times of from 2 to 10 minutes are used. The sanitizing step is preferably followed by a final rinse with hot or cold water.

In many applications, the above-described process has been found suitable for continuous sanitizing processes. Then, during immediate direct delivery from the reactant supply (normally hydrogen peroxide, formic acid, catalyst) to the surface of application, mixing of the reactants to form the performic acid, takes place simultaneously with or just prior to dilution to the required concentration. In such continuous applications of performic acid, preferably reaction temperatures are around ambient temperature, or still more preferably within the range of 40 to 70°C, resulting in quick formation of performic acid after the mixing of the reactants.

Another aspect of the present invention comprises a 'kit' for effecting the processes related above. This kit is essentially a set of reactants and, optionally, a catalyst for preparing the performic acid. In one preferred form it comprises:-

(i) a container of hydrogen peroxide as from 10 to 50% by weight in aqueous solution;

(ii) a container of formic acid as from 5 to 100% by weight in aqueous solution;

(iii) a catalyst capable of catalysing the reaction of hydrogen peroxide with formic acid and to form performic acid, said catalyst being in admixture with either the solution in container (i) or the solution in container (ii), or both, and wherein the volumes of liquid in containers (i) and (ii) are such that the weight concentration ratio between the hydrogen peroxide per se and the formic acid per se, is in the range of from 1:6 to 1:1.5. Preferably, the kit is also presented together with instructions for mixing the reactants, if necessary with details of appropriate reaction temperatures, reaction times, application concentrations and temperatures, and storage capabilities. The kit may also comprise a stabilizer for prolonging the storage life of the thus prepared performic acid.

The invention is now further illustrated by way of examples. As used hereinbelow, 'CMCC' is the designation of culture collection number of the Unilever Research Colworth Laboratory, Sharnbrook. Bedfordshire, England.

## In situ preparation of performic acid

A total of 1600 ml of reaction mixture was prepared by mixing 1000 ml of a 30% by weight hydrogen peroxide solution and 600 ml of a 50% by weight formic acid solution which included 28 grams of sulphuric acid.

The hydrogen peroxide and formic acid solutions were dosed into a reaction vessel simultaneously over a period of 90 seconds by two peristaltic pumps, the speeds of which were adjusted such that the dosing time for each of the solutions was the same.

Equilibrium was reached in about 30 minutes, resulting in a performic acid concentration of about 2.5% by weight.

Performic acid solutions of different concentrations were also prepared by substantially the same method but using different concentrations of hydrogen peroxide and/or formic acid. In the following description, deviations from the above preferred concentrations are indicated where relevant.

To illustrate the sanitizing effectiveness of performic acid, various test organisms were used, the preparations of which is described hereunder.

## Bacterial spore suspensions

Spore crops were prepared for each organism by inoculating the surface of each of 8 plastic trays - (measuring approx. 12" $\times$ 10", each containing 420 ml plate count agar) with 5 ml of a pasteurised spore suspension. The inoculated trays were incubated at 37°C for 3 days, after which time only phase-bright spores and no vegetative cells were visible under phase contrast microscopy.

The spores from all 8 trays were scraped from the surface of the agar, using a glass slide, and were combined. The pooled spores were then washed 9 times in distilled water with intermediate centrifugation at 10K rpm for 20 minutes at 4°C. The final pellet was resuspended in distilled water to give approximately $10^9$ spores/ml (using a value of $A_{540}$ of 0.35 equivalent to $10^7$ spores/ml). This suspension was dispensed in 2 ml volumes into sterile bijou bottles which were stored at -20°C.

When required for use, the individual bijous were thawed at room temperature and then pasteurised at 75°C for 30 minutes before diluting (1:100) to give approximately $10^7$ spores/ml.

The organisms for which crops were prepared in this way were:

Bacillus cereus     CMCC 629
Bacillus subtilis     CMCC 604
Bacillus subtilus     SA22
Bacillus polymyxa     B36

Bacterial Cultures

There were prepared from freeze-dried ampoules and were revived and maintained using the conditions shown in Table 1.

## Table 1

### Incubation conditions for vegetative cultures

| Organism | CMMC Number | Temp °C | Medium | Days Incubation |
|---|---|---|---|---|
| Escherichia coli | 1585 | 37 | NB/NA | 1 |
| Staphylococcus aureus | 295 | 37 | NB/NA | 1 |
| Pseudomonas fluorescens | 2882 | 30 | NB/NA | 1 |
| Bacillus polymyxa B36 | | 30 | NB/NA | 1 |
| Streptococcus faecalis | 2834 | 37 | NB/NA | 1 |
| Mycobacterium phlei | 820 | 37 | HIB/HIA | 2 |
| Acetobacter aceti subsp. aceti | 3000 | 25 | NB/NA | 3 |
| Saccharomyces rouxii | 2284 | 25 | MEB/MEA | 2 |
| Saccharomyces cerevisiae | 456 | 25 | MEB/MEA | 2 |

NB/NA     : Nutrient broth/agar
HIB/HIA    : Heart infusion broth/agar
MEB/MEA   : Malt extract broth/agar

Dairy bacteriophage

A freeze-dried ampoule of Streptococcus cremoris (NIRD 924) and its homologous phage were obtained from NIRD, Shinfield, Reading. The culture was revived and phage stocks prepared according to the method of Mullen et al, Journal of Dairy Res. 48, 465-471.

## Fungal spore crops

Two fungi (Aspergillus terreus CMCC424 and Penicillium sp CMCC250) were used in the experiments. The fungal spores were washed from potato/dextrose/agar surfaces with sterile distilled water containing a trace of Tween 80. The spore crops were stored at 4°C, and at use diluted to 1:100.

## Measurement of antimicrobial activity

Sporicidal activity was measured using contact bottles containing a total of 10ml spore suspension plus peracid solution. After various contact times, a 0.1 ml aliquot was added to 10 ml quenching agent. Spread plates were prepared from decimal dilutions thereof prepared in 0.1% peptone.

The quenching agent was 1% (w/v) ascorbic acid in double strength McIlvaine buffer (citrate-$Na_2HPO_4$) at pH 7.0.

Antibacterial activity was measured using broth cultures that were centrifuged once (5000 g, 10 min. 10°C), resuspended to the original volume in sterile distilled water and dispensed in 10 ml volumes in 1 oz Macartney bottles. Aliquots of test-solution performic acid were subsequently added to 10 ml phage stock suspension in the Macartney bottles. After various contact times, a 1 ml sample of the contact bottle was added to 9 ml quenching agent. Enumeration of the bacteriophage (both inoculum and survivors) was achieved using the soft overlay technique for plaque counts, using the media of Mullen et al. Plates were incubated at 30°C for 48 h before plaques were counted.

Activity results are expressed as "log reduction" which denotes the $^{10}$log of the reduction in surviving organisms:

$$Log\ reduction = {}^{10}log\ \left(\frac{initial\ organisms}{surviving\ organisms}\right)$$

In Table 2 activity results are presented obtained with highly diluted performic acid solutions. The prepared performic acid solution was made in the way described above (30% hydrogen/50% performic acid) and diluted to the working concentrations as indicated in Table 2, 30 minutes after preparation. The working concentrations correspond to performic acid concentrations of 2.5 ppm (0.01%) to 12.5 ppm (0.05%).

## Table 2

### Activity of dilute performic acid against Escherichia coli

| working concentration | log reduction after contact time of:- | | | |
|---|---|---|---|---|
| | 10 sec | 20 sec | 30 sec | 60 sec |
| 0.01% | 0.1 | | 0.3 | 0.4 |
| 0.015% | 0.4 | 0.7 | 0.9 | 1.2 |
| 0.02% | 0.6 | 1.2 | 1.9 | 3.1 |
| 0.05% | 4.6 | >5.0 | >5.0 | >5.0 |

From Table 2 it is clear that at very dilute concentrations, significant sanitizing effects are obtained within short periods of time. Higher concentrations of performic acid are required to kill bacterial spores - (Table 9) but the activity of performic acid is clearly superior to that of other peroxygen disinfectants (e.g. Table 10).

In Table 3 the same in situ prepared performic acid solution was tested against a number of test organisms at two working concentrations corresponding to performic acid concentrations of 12.5 ppm and 250 ppm. It is evident that performic acid is active against a broad range of micro-organisms and their spores.

## Table 3

### Activity of performic acid against a range of microorganisms

| Organism | CMCC number | Time to 4 log reduction (working concentration) | |
| --- | --- | --- | --- |
| | | 0.05% v/v | 1.0% v/v |
| Vegetative cells | | | |
| Staphylococcus aureus | 295 | 20 sec | |
| Pseudomonas fluorescens | 2882 | 10 sec | |
| Escherichia coli | 1585 | 10 sec | |
| Streptococcus faecalis | 2834 | 20 sec | |
| Mycobacterium phlei | 820 | 1 min | |
| Acetobacter aceti subsp. aceti | 3000 | 30 sec | |
| Bacillus cereus | 629 | 10 sec | |
| Bacillus polymyxa B36 | | 10 sec | |
| Saccharomyces cerevisiae | 456 | 10 min | 20 sec |
| Saccharomyces rouxii | 2284 | | 1 min |
| Spores | | | |
| Aspergillus terreus | 424 | | 1 min |
| Penicillium sp. | 2501 | | 5 min |
| Bacillus cereus | 629 | | 5 min |
| Bacillus subtilis | 604 | | 5 min |
| Bacillus polymyxa B36 | | | 5 min |
| Bacillus subtilis SA22 | | | 3 min |
| Bacteriophage | | | |
| Phage 0924 (Streptococcus cremoris) | | | 1 min |

8

In Tables 4 and 5 activity results are presented for a variety of formic acid/hydrogen peroxide reaction mixtures at two temperatures. Working concentrations of 1% were used, reaction mixture being diluted to working concentrations after 15 minutes. The test organism was Bacillus cereus. Contact times of 15 minutes were used.

## Table 4

### Sporicidal activity of performic acid at 8°C

|  |  | % Formic acid:- | | | |
|---|---|---|---|---|---|
|  |  | 0 | 10 | 25 | 50 |
|  | 0 | 0.0 | 0.0 | 0.0 | 0.0 |
| % hydrogen | 10 | 0.2 | 0.3 | 0.8 | 0.4 |
| peroxide | 20 | 0.1 | 0.6 | 1.1 | 2.6 |
|  | 30 | 0.2 | 0.8 | 1.6 | 3.8 |

## Table 5

### Sporicidal activity of performic acid at 45°C

|  |  | % Formic acid:- | | | | |
|---|---|---|---|---|---|---|
|  |  | 0 | 5 | 10 | 25 | 50 |
|  | 0 | 0.0 | 0.0 | 0.2 | 0.0 | 0.0 |
| % hydrogen | 10 | 0.1 | 0.2 | 0.4 | 1.2 | 3.1 |
| peroxide | 20 | 0.1 | 0.3 | 0.6 | 1.7 | 3.4 |
|  | 30 | 0.0 | 0.4 | 0.9 | 3.1 | 4.3 |

In Table 6 results are presented for a 50% formic acid -30% hydrogen peroxide mixture which was diluted after 30 minutes to the working concentrations indicated. The working concentrations correspond to performic acid concentrations of 25 ppm (0.1%) to 0.125% (5.0%). Performic acid is active over a wide range of temperatures but its efficacy is enhanced with increasing temperature and concentration.

## Table 6

### Sporicidal activity of performic acid at different temperatures

| working concentration (% v/v) | log reduction after 10 seconds contact:- | | | |
|---|---|---|---|---|
| | 20°C | 40°C | 60°C | 80°C |
| 0.1 | | | 0.5 | 0.3 |
| 0.3 | | | 0.6 | |
| 0.5 | | | 1.3 | 2.3 |
| 1.0 | 0.7 | 0.9 | 4.3 | 4.3 |
| 3.0 | 1.5 | 2.3 | 4.3 | 4.3 |
| 5.0 | 4.3 | 4.3 | 4.3 | 4.3 |

In Table 7 results are presented showing the effects on sporicidal activity of varying hydrogen peroxide/formic acid mixtures and varying periods of time between preparation of the reaction mixture and dilution to working concentration.

## Table 7

### Sporicidal of diluted performic acid

| % hydrogen peroxide | % formic acid | pre-contact dilution time in min:- | | | | | |
|---|---|---|---|---|---|---|---|
| | | 0 | 5 | 15 | 60 | 120 | 180 |
| 30 | 25 | 4.1 | 3.7 | 3.0 | 3.1 | 2.2 | 2.0 |
| 30 | 10 | 1.1 | 1.1 | 1.0 | 0.9 | 0.4 | 0.4 |
| 30 | 5 | 0.4 | 0.9 | 0.7 | 0.5 | 0.4 | 0.2 |
| 30 | 50 | 4.1 | 4.1 | 4.1 | 4.1 | 3.4 | 3.7 |
| 20 | 50 | 4.1 | 4.1 | 4.1 | 4.1 | 3.6 | 2.5 |
| 10 | 50 | 2.6 | 2.7 | 2.7 | 2.2 | 1.4 | 1.1 |

In Table 8 results are presented showing the effects on sporicidal activity of varying hydrogen peroxide/formic acid mixtures and varying contact times.

## Table 8

### Effect of contact time on sporicidal activity of performic acid

| % hydrogen peroxide | % formic acid | contact time (min):- | | | | |
|---|---|---|---|---|---|---|
| | | 1 | 5 | 10 | 15 | 30 |
| 0 | 50 | 0.0 | 0.0 | 0.1 | 0.0 | 0.0 |
| 10 | 50 | 0.4 | 2.1 | 4.1 | 4.1 | 4.1 |
| 20 | 50 | 0.2 | 2.8 | 4.1 | 4.1 | 4.1 |
| 30 | 50 | 0.9 | 4.1 | 4.1 | 4.1 | 4.1 |
| 30 | 0 | 0.0 | 0.0 | 0.0 | 0.0 | 0.0 |
| 30 | 5 | 0.0 | 0.3 | 0.3 | 0.4 | 0.7 |
| 30 | 10 | 0.0 | 0.4 | 0.9 | 1.1 | 2.1 |
| 30 | 25 | 0.5 | 1.5 | 3.5 | 4.1 | 4.1 |

In Table 9 sporicidal activities are presented for working concentrations of a 50% formic acid/30% hydrogen peroxide reaction mixture which was diluted 30 minutes after preparation. Contact times were 5 minutes. Contact temperature was 20°C.

## Table 9

### Sporicidal activity of diluted performic acid

| in-use concentration (%) | 0.1 | 0.3 | 0.5 | 0.7 | 1.0 |
|---|---|---|---|---|---|
| corresponding to a performic acid concentration (ppm) | 25 | 75 | 125 | 175 | 250 |
| log reduction of B. cereus spores | 0.0 | 1.9 | 4.4 | 4.4 | 4.4 |

## Table 10

### Comparison of peroxygen disinfectants

% (w/v) giving 1 log reduction in viable
count of Bacillus cereus spores in 1 minute:-

| Temperature °C | Hydrogen peroxide | Peracetic acid | Performic acid |
|---|---|---|---|
| 20 | 30 | 0.48 | 0.0125 |
| 60 | 7.5 | 0.10 | 0.0025 |

**Claims**

1. The industrial use of performic acid as a sanitizer.

2. The industrial use of performic acid as claimed in claim 1, when in the food industry.

3. The industrial use of performic acid as claimed in claim 2, when in the dairy industry.

4. The industrial use of performic acid as claimed in claim 3, to sanitize ultra high temperature milk sterilization equipment.

5. The industrial use of performic acid as claimed in claim 2, when in the brewing industry.

6. The industrial use of performic acid as claimed in claim 5, to sanitize bright beer tanks.

7. Industrial use of performic acid as claimed in any of claims 2-6, as part of a cleaning-in-place process.

8. The industrial used of performic acid as claimed in claim 1, to sterilize asceptic packaging.

9. An industrial process for sanitizing a surface, characterised by preparing performic acid and applying it at an effective concentration to the surface to be sanitized, before, during or after an industrial operation.

10. An industrial process as claimed in claim 9, further characterised in that the industrial operation is a manufacturing, processing or packaging operation.

11. An industrial process as claimed in claim 9 or claim 10, further characterised in that the performic acid is applied to the surface simultaneously with, or within a relatively short period after, initiating the preparation.

12. An industrial process as claimed in claim 11, further characterised in that the time between initiating the preparation and first applying the solution to the surface is in the range of from 15 minutes to 3 days.

13. A method according to claim 12, further characterised in that the time is in the range of from 15 minutes to 48 hours.

14. An industrial process as claimed in any of claims 9-13 further characterised in that the performic acid is prepared on the site where the surface to be sanitized is located.

15. An industrial process as claimed in claim 14, further characterised in that the performic acid is prepared in a reaction vessel located adjacent to the surface to be sanitized.

16. An industrial process as claimed in claim 15, further characterised in that after mixing of reactants to initiate preparation, the performic acid is applied substantially immediately via a direct link from the reaction vessel to said surface.

17. An industrial process as claimed in claim 15, further characterised in that after preparation, the performic acid is stored in the reaction vessel, prior to application to the surface to be sanitized.

18. An industrial process as claimed in claim 15, further characterised in that after preparation, the performic acid is stored in a separate storage vessel, prior to application to the surface to be sanitized.

19. An industrial process as claimed in any of claims 9-18, further characterised by comprising the step of removing the performic acid from the surface, subsequent to application.

20. An industrial process according to any of claims 9-19, further characterised in that the performic acid solution is prepared at a concentration in the range of from 2 to 8% by weight.

21. An industrial process according to any of claims 9-20, further characterised in that the concentrations is in the range from 3 to 7% by weight.

22. An industrial process according to any of claims 9-21, further characterised in that the preparation of the performic acid comprises the steps:-

(i) reacting hydrogen peroxide or a solution thereof, with formic acid or a solution thereof; and if necessary to achieve the desired effective concentration in solution -

(ii) diluting the reaction product with water.

23. An industrial process according to any of claims 9-22, further characterised in that the performic acid is applied to the surface at a concentration in the range of from 2 to 300 ppm.

24. An industrial process according to claim 23, further characterised in that the applied concentration is in the range of from 30 to 150 ppm.

25. An industrial process according to any of claims 9-24, further characterised in that the performic acid is applied to the internal surface of equipment as part of a cleaning-in-place operation.

26. An industrial process according to claim 25, further characterised in that the performic acid is applied to the internal surface, simultaneously with, or subsequent to application of another substance to the surface.

27. An industrial process according to claim 26, further characterised in that the other substance is a surfactant.

28. An industrial process according to any of claims 25-27, further characterised in that the performic acid is applied to the surface substantially at the working temperature of the equipment.

29. An industrial process according to any of claims 25-28, further characterised in that the contact time of the performic acid on the surface is in the range of from 1 to 30 minutes.

30. An industrial process according to claim 29, further characterised in that the contact time is in the range of from 2 to 10 minutes.

31. A preparation for use as claimed in any of claims 1-8, comprising:-

(i) a container of hydrogen peroxide as from 10 to 50% by weight in aqueous solution;

(ii) a container of formic acid as from 5 to 100% by weight in aqueous solution;

(iii) a catalyst capable of catalysing the reaction of hydrogen peroxide with formic acid to form performic acid, said catalyst being in admixture with either the solution in container (i) or the solution in container (ii), or both, and wherein the volumes of liquid in containers (i) and (ii) are such that the weight concentration ratio between the hydrogen peroxide per se and the formic acid per se is in the range of from 1:6 to 1:1.5.